# EUROPÄISCHE PATENTSCHRIFT

(11) **EP 1 536 754 B1**
(45) Veröffentlichungstag und Bekanntmachung des Hinweises auf die Patenterteilung: **20.05.2015**
(21) Anmeldenummer: 03794849.4
(22) Anmeldetag: 07.08.2003
(51) Int. Cl.: A61K 8/97, A61Q 1/02, A61K 8/92

(54) **LIPIDHALTIGE ZUBEREITUNG, INSBESONDERE KOSMETISCHE ZUBEREITUNG**
LIPID PREPARATION, PARTICULARLY COSMETIC PREPARATION
PREPARATION LIPIDIQUE, NOTAMMENT PREPARATION COSMETIQUE

(30) Priorität: 31.08.2002 DE 10240322
(43) Veröffentlichungstag der Anmeldung: 08.06.2005
(73) Patentinhaber: Schwan-STABILO Cosmetics GmbH & Co. KG, 90562 Heroldsberg (DE)
(72) Erfinder: LEBOK, Simona, 90409 Nürnberg (DE); WINKLER, Wolfgang, 91207 Lauf (DE)
(74) Vertreter: Leissler-Gerstl, Gabriele
(86) Internationale Anmeldenummer: PCT/EP2003/008793
(87) Internationale Veröffentlichungsnummer: WO 2004/024105

(56) Entgegenhaltungen:
- WO-A-02/38109
- DE-U- 20 213 416
- GB-A- 1 134 170
- US-A- 4 871 536
- US-A- 5 800 818
- US-B1- 6 180 668
- PATENT ABSTRACTS OF JAPAN & JP 2000 297013 A (NARIS COSMETICS CO LTD), 24. Oktober 2000 (2000-10-24)
- ARQUETTE J ET AL: "JOJOBA ESTERS IN LIPSTICKS", COSMETICS & TOILETRIES, WHEATON, IL, US, vol. 113, no. 7, 1 July 1998 (1998-07-01), pages 63-65,67, XP001117577, ISSN: 0361-4387
- "Jojoba" In: Prof. Dr. Jürgen Falbe,Pr.Dr.Manfred Regitz: "RÖMPP CHEMIE LEXIKON", 1995, Georg Thieme Verlag, Stuttgart-New York page 210, * Jojoba; page 210 *

## Beschreibung

Die Erfindung betrifft eine lipidhaltige Zubereitung gemäß Anspruch 1, insbesondere in Form eines Stiftes oder einer geschmeidigen Paste, welche sich für kosmetische Anwendungen, insbesondere im Bereich der dekorativen Kosmetik, zum Färben und Verschönen der Haut, der Lippen und der Augenlider, eignet. Beispielhaft genannt seien hier Lippen- und Wangenrouge, Lipliner, Eyeliner, Augenbrauenstifte, Make-up, Abdeckstift, Concealer oder Lidschatten. Sie kann auch als Lippenpflegestift, Lippenbalsam, Lipgloss, als fixierende Grundlage für die Lippen, als Pflegegrundlage zur Pflege der Haut oder als Sonnenschutzmittel verwendet werden. Diese lipidhaltige Zubereitung liegt in wasserfreier Form vor.

Zubereitungen der genannten Art enthalten üblicherweise Lipide, wie z.B. Fette, Öle, öllösliche Pflanzenauszüge und mittel- bis langkettige Fettsäuren und Wachse, welche pflanzlichen oder tierischen Ursprungs sein können, sich von mineralischen Quellen, wie z.B. dem Erdöl, ableiten oder durch Synthese oder chemische Veränderung der genannten Stoffe erhalten wurden. Bekannt ist auch die Verwendung von siliciumorganischen Verbindungen, wie z.B. Dimethicone, Phenyltrimethicone, Diphenyldimethicone, flüchtigen Cyclomethiconen, Siliconwachsen und dergleichen.

Daneben kann eine feste Phase enthalten sein, welche aus feinteiligen Füllstoffen und Färbemitteln besteht. Im Falle von Sonnenschutzmitteln können besonders feinteilige Pigmente, sog. Nanopigmente mit einer durchschnittlichen Teilchengröße zwischen 5 und 25 nm, Anwendung finden, welche auf der Haut transparent wirken und sie nicht mehr einfärben. Beispielhaft genannt seien hier Siliciumdioxid, Titandioxid und Zinkoxid.

Von Erdöl abgeleitete Produkte stehen nicht unbegrenzt zur Verfügung, da sie aus endlichen Quellen stammen. Zudem gibt es Empfehlungen aus dermatologischer Sicht, z.B. von der SCF, im Lippenbereich vorsorglich keine erdölbasierten Rohstoffe einzusetzen, um deren Anreicherung im Magen-Darmtrakt zu vermeiden. Daher sind pflanzliche Rohstoffe, welche nachwachsen, diesen vorzuziehen. Bei pflanzlichen Rohstoffen stellen sich auch die Fragen nicht, die sich aus einer Massentierhaltung mit anschließender Nutzung von Tieren ergeben.

Aus DE 199 40 221 ist z.B. ein Körperpflegemittel bekannt, das in Form eines freistehendes Stiftes gebildet ist. Damit der Stift seine Form behält, muss er einen ganz speziellen Aufbau haben, wobei er einen Innenkern mit kosmetischen Bestandteilen aufweist und von einer Schicht umhüllt ist, die aus Proteinen, Polyolen, Erweichungsmitteln und ggf. kosmetischen Hilfsstoffen aufgebaut ist. Durch den vorgegebenen Aufbau sind mehrere Arbeitsschritte zur Herstellung erforderlich.

Aus DE 107 07 309 ist eine feste kosmetische Zusammensetzung bekannt, die aus verschiedenen verfestigten kosmetischen Ölen aufgebaut ist. Dazu werden in den Beispielen Mischungen von tierischen und synthetischen Ölen eingesetzt. Nachteil dieser Zusammensetzung ist es, dass nur sehr geringe Mengen an Pigmenten verwendet werden können, die in einem Bereich von nur 0,1 bis 4 Gew.-% liegen.

Das deutsche Patent DE 304 76 49 beschreibt eine kosmetische Zusammensetzung in Emulsionsform, die aus einer Mischung von Ölen und dem nicht verseifbaren Bestandteil eines Öls besteht. Die Zusammensetzung ist weder dazu gedacht und noch dazu geeignet, als Stift oder Paste formuliert zu werden.

In der europäischen Patentanmeldung EP 0 667 146 hatten sich die Erfinder die Aufgabe gestellt, eine Lippenschminke in Form einer weichen Paste, die mittels Applikator auftragbar ist, bereitzustellen und lösten diese Aufgabe durch Bereitstellung einer Zusammensetzung, die ein Wachs in einer Fettphase enthält, wobei die dynamische Viskosität in einem vorbestimmten Bereich eingestellt wird. Bevorzugte Fettbestandteile sind dabei aus Erdöl stammende Komponenten, wie Paraffinöl, Vaseline, Mineralöl, deren Verwendung mit der vorliegenden Erfindung gerade vermieden werden soll.

Eine Literaturstelle, die sich mit einer wasserhaltigen Rezeptur für einen Lippenstift befasst, ist EP 0 522 618. Nachteil bei wasserhaltigen Rezepturen ist die höhere Anfälligkeit für eine bakterielle Kontamination und damit die geringere Stabilität.

Eine Zusammensetzung aus einer Ölphase und einem Pigment ist aus US-6,013,122 bekannt. Hier handelt es sich allerdings um eine flüssige Tattoo-Tinte, die nicht dauerhaft anfärbt, sondern entfernbar ist. Diese Zusammensetzung ist nicht dazu vorgesehen, zu Stiften oder Pasten verarbeitet zu werden.

Um ein Öl der Verarbeitung in kosmetischen Massen zugänglich zu machen, ist ein Raffinierungsverfahren erforderlich. Eine spezielle Ausführungsform hierzu wird in US-5,653,966 beschrieben.

Eine an mehrfach ungesättigten Fettsäuren reiche Zusammensetzung ist aus US-5,445,822 bekannt, die zu einem kosmetischen Präparat verarbeitet werden soll, insbesondere einer Emulsion. Nachteil einer solchen Zusammensetzung ist ihre geringe Stabilität.

Die Erfinder von WO 02/38109 haben herausgefunden, dass durch superkritische Fluidextraktion aus Rubus Chamaemorus Bestandteile extrahiert werden können, die als Zusatz zu Kosmetikzusammensetzungen vorteilhaft sind. Die Herstellung derartiger kosmetischer Produkte ist jedoch ziemlich aufwändig.

Bei der Herstellung und Verarbeitung von lipidhaltigen Zubereitungen der genannten Art ist zu berücksichtigen, dass sie über längere Zeit höheren Temperaturen im Bereich um 70 bis 110 °C ausgesetzt sein können - zum einen, wenn die Rohstoffe aufgeschmolzen und zusammen mit Färbemitteln und sonstigen Zusätzen homogenisiert werden und zum anderen, wenn sie in ihre endgültige Form gebracht werden. Die verwendeten Rohstoffe dürfen sich dabei durch die Einwirkung von Hitze und Luftsauerstoff nicht oder nur in sehr geringem Umfang chemisch verändern. Pflanzliche Rohstoffe, insbesondere dann wenn sie Doppelbindungen oder konjugierte Doppelbindungen in der Kohlenstoffkette enthalten, neigen zu Umlagerungen, Anlagerungen, Peroxidbildung und dergleichen, welche zu negativen geruchlichen Veränderungen führen können. Durch die Anwesenheit bestimmter Pigmente können solche Veränderungen induziert oder auch im Sinne einer Katalysatorwirkung beschleunigt werden. Bisher sind nur wenige pflanzliche Rohstoffe bekannt, die mit einem befriedigenden Ergebnis und ohne Qualitätseinbußen zu lipidhaltigen Zubereitungen der genannten Art verarbeitet werden können.

Aufgabe der Erfindung war es daher, eine wasserfreie lipidhaltige Zubereitung, insbesondere in Form eines Stiftes oder einer geschmeidigen Paste, welche sich für kosmetische Anwendungen, insbesondere im Bereich der dekorativen Kosmetik, zum Färben und Verschönen der Haut, der Lippen und der Augenlider eignet, die sich leicht auftragen lässt, gut haftet und lange hält, und die vom ursprünglichen Auftragsort nicht oder nur minimal in die unmittelbare Umgebung einwandert, bereitzustellen. Diese Zubereitung soil auf rein pflanzlichen und pflanzenbasierten Rohstoffen aufgebaut sein, welche die vorgenannten Nachteile nicht aufweisen, ansonsten aber möglichst frei sein von tierischen Rohstoffen, erdölbasierten Rohstoffen, insbesondere, um deren bekannte Nachteile im unmittelbaren Augenbereich zu vermeiden. Außerdem soli es möglich sein, auf den Zusatz von Siliconölen oder Siliconderivaten zu verzichten. Die Zubereitung soli - wenn sie in Stiftform vorliegt - bei unterschiedlichen Aufbewahrungstemperaturen, die sich auf den unterschiedlichen Transportwegen und beim Anwender selbst ergeben können, lagerstabil sein und - wenn sie als geschmeidige Paste vorliegt - keine Synäreseeffekte nach längerer Lagerung zeigen.

Diese Zubereitung soli sich also im Vergleich zu Produkten nach dem Stand der Technik weich und geschmeidig auftragen lassen, auf der Haut, den Lidern und den Lippen nicht spannen und diese nicht austrocknen, gut und anhaltend haften, sich möglichst nicht auf Gegenstände und Textilien oder andere Hautbereiche übertragen und in unmittelbarer Augennähe auch nicht zu Reizungen oder anderen negativen Empfindungen führen.

Die Aufgabe wird dadurch gelöst, dass eine wasserfreie lipidhaltige Zubereitung bereitgehalten wird, welche ausschließlich aus pflanzlichen oder pflanzenbasierenden Rohstoffen aufgebaut ist, bei denen es sich wenigstens teilweise um hydrierte pflanzliche oder pflanzenbasierte Rohstoffe handeln soil.

Überraschenderweise wurde gefunden, dass die erfindungsgemäße Zubereitung aufgrund ihrer Zusammensetzung dazu geeignet ist, selbst bei Pigmentgehalten von 40 bis 50 Gew.-% zu Minen gegossen zu werden, so dass aus der erfindungsgemäßen Masse in einfacher Weise Stifte hergestellt werden können: Dies war mit den bekannten Massen unter Anwendung pflanzlicher Bestandteile bisher nicht möglich. Außerdem werden erfindungsgemäß besonders stabile Zubereitungen bereitgestellt.

Die Aufgabe wird durch die in Anspruch 1 definierte wasserfreie lipidhaltige Zubereitung gelöst.

Inbesondere wird erfindungsgemäße eine wasserfreie lipidhaltige Zubereitung bereitgestellt, die mindestens eine Ölphase und eine Feststoffphase aufweist. Dabei ist die Ölphase ausschließlich aus pflanzlichen oder pflanzenbasierten Rohstoffen aufgebaut und enthält Hydrogenated Jojoba Oil und Limnanthes Alba (Meadowfoam Seed Oil).

Die Bezeichnung der Rohstoffe erfolgt mit den den einschlägig befassten Fachleuten bekannten Bezeichnungen gemäß der "International Nomenclature of Cosmetic Ingedients" (sog. "INCI-Namen"). Beispielhaft seien hier genannt: hydrierte Pflanzenöle wie z.B. Hydrogenated Jojoba Oil, Hydrogenated Cottonseed Oil, Hydrogenated Vegetable Oil, Hydrogenated Rapeseed Oil, Hydrogenated Castor Oil, Hydrogenated Coco-Glycerides und ähnliche, sowie Magnifera Indica (Mango Seed Oil), Limnanthes Alba (Meadowfoam Seed Oil), Butyrosperum Parkii (Shea Butter), Macadamia Ternifolia Nut Oil (Macadamia Nut Oil), Buxus Chinensis (Jojoba Oil); Wachse wie Carnauba, Candelillawachs, Japanwachs (Rhus Succedanea), Reiswachs, Zuckerrohrwachs und ähnliche.

Der Gehalt an hydrierten Pflanzenölen in der lipidhaltigen Zubereitung liegt dabei zwischen 5 und 80 Gew.-%, vorzugsweise zwischen 15 und 50 Gew.-%. Der Gehalt an pflanzlichen Wachsen beträgt 0,1 bis 30 Gew.-%, vorzugsweise 1 bis 20 Gew.-%. Der Gehalt an pflanzlichen Ölen, einschließlich Jojobaöl (Buxus Chinensis), bei dem es sich im chemischen Sinne um ein flüssiges Wachs handelt, liegt in einem Bereich von 1 bis 65 Gew.-%, vorzugsweise bei 5 bis 45 Gew.-%. Die Angaben der eingesetzten und vorzugsweise eingesetzten Mengen erfolgen natürlich mit der Maßgabe, dass sich ihre Summe schließlich auf 100 Gew.-% ergänzt.

Als besonders vorteilhaft hat sich eine lipidhaltige Zubereitung der genannten Art erwiesen, welche eine Kombination aus Hydrogenated Jojoba Oil und Limnanthes Alba (Meadowfoam Seed Oil) enthält. Hydrogenated Jojoba Oil ist im Handel erhältlich und ist in verschieden hohen Hydrierungsgraden und damit mit unterschiedlicher Härte - gemessen bspw. durch Nadel-penetration - und mit unterschiedlich hohem Schmelzpunkt erhältlich. Durch entsprechendes Abmischen verschiedener im Handel erhältlicher Produkttypen, kann der einschlägig befasste Fachmann die für seine Zwecke am besten geeignete Mischung herausfinden und so gegebenenfalls Endprodukt optimal, z.B. bezüglich Konsistenz und Viskosität einstellen.

Die Einsatzmengen von Hydrogenated Jojoba Oil und Limnanthes Alba (Meadowfoam Seed Oil) sollen dabei jeweils zwischen 2 und 35 Gew.-%, vorzugsweise zwischen 5 und 25 Gew.-% liegen und zueinander in einem Verhältnis zwischen 1 : 2 bis 2 : 1 eingesetzt werden. Limnanthes Alba (Meadowfoam Seed Oil) gilt als besonders oxidations- und ranzditätsstabiles pflanzliches Öl - diese Produkteigenschaft konnte durch die Kombination mit Hydrogenated Jojoba Oil noch gesteigert werden, so daß die erfindungsgemäßen Zubereitungen sich als überraschend lagerstabil, auch bei erhöhten Lagertemperaturen zeigten. Sie sind aber nicht nur sehr oxidationssatbil sondern verändern auch über eine längere Lagerzeit die olfaktorischen Eigenschaften nicht. Die erfindungsgemäßen lipidhaltigen Zubereitungen sind bis in Temperaturbereiche von etwa 42 °C unverändert anwendbar und bis etwa 52 °C lagerstabil. Sie sind zudem leicht, geschmeidig und gleichmäßig auftragbar, gut haftend und lange haltbar, ohne auf der Haut in einem wesentliche Umfang zu spreiten. So ist es beispielsweise auch möglich, aus einer lipidhaltigen Zubereitung der genannten Art kosmetische Stifte mit gegossenen Minen herzustellen, welche einen Minendurchmesser im Bereich von 2 bis 6 mm aufweisen können, mit einem Pigment- und Feststoffgehalt in einem Bereich von 1 bis etwa 50 Gew.-%, bevorzugt 5 bis 40 Gew.-%, ganz besonders bevorzugt 10 bis 30 Gew.-%, bezogen auf die Gesamtmenge der Zubereitung. Solche kosmetischen Stifte, die bei Pigmentgehalten um 40 bis 50 Gew.-%, bisher vorzugsweise durch ein Extrusionsverfahren hergestellt wurden, finden wegen ihres guten Deckvermögens insbesondere Verwendung als Lipliner, Eyeliner oder Augenbrauenstifte. Durch den Einsatz der vorgenannten Kombination aus Hydrogenated Jojoba Oil und Limnanthes Alba (Meadowfoam Seed Oil) werden lipidhaltige Zubereitungen der genannten Art mit hohen Feststoffgehalten bis 50 Gew.-% den modernen Gießverfahren zugänglich - grundsätzlich ist es aber möglich, solche lipidhaltige Zubereitungen auch nach den bekannten Verfahren zu extrudieren und anschließend mit den ebenfalls bekannten Verfahren zu holzgefassten Kosmetikstiften weiterzuverarbeiten.

Der zweite wesentliche Bestandteil der erfindungsgemäßen Zubereitung ist eine Feststoffphase, die aus Füllstoffen und/oder Pigmenten bestehen kann. Der Anteil der festen Phase kann je nach gewünschter Konsistenz und gewünschter Wirkung der Masse eingestellt werden. So richtet sich der Anteil des Pigments u.a. nach der gewünschten Farbe und der Anteil an Füllstoffen nach der gewünschten Konsistenz der Masse. Auch Effektmittel, wie glitzernde oder leuchtende Bestandteile können eingearbeitet werden.

Beispiele für Stoffe, die die feste Phase bilden können, sind beispielsweise Füllstoffe, wie z.B. Talkum, Kaolin, Stärke und modifizierte Stärke, Polytetrafluorethylenpulver (Teflon), Nylonpulver, Bornitrid, aus unlöslichen Metallseifen, wie Mg-Stearat, Ca-Stearat, Sr-Stearat, Zn-Stearat und aus anorganischen oder organischen Pigmenten bestehen. Für letztere seien beispielhaft genannt: Titandioxid, Zinkoxid, Eisenoxide, Chromoxid, Chromoxidhydrat, Ultramarin, Berliner Blau (Ferric Blue), Glimmer, Perlglanzmittel wie z.B. mit Titandioxid beschichtete Glimmer, farbige, mit Titandioxid und Metalloxiden beschichtete Glimmer, Bismuthoxidchlorid, beschichtetes Bismuthoxidchlorid, plättchenförmige Metallpulver von Aluminium, Messing, Bronze, Kupfer, Silber, Gold, sowie Verlackungen organischer Färbemittel mit Aluminium, Barium, Calcium oder Strontium. Diese Aufzählung ist nur beispielhaft und nicht abschließend. Diese Zusätze erfolgen mit der Maßgabe, daß sie von der jeweiligen nationalen oder regionalen Kosmetik-Gesetzgebung auch zugelassen sind.

Die Menge der festen Phase richtet sich bevorzugt nach der in den einzelnen Ländern durch Kosmetikgesetzgebung geregelten Höchstmenge.

Die Mengenanteile an Pigmenten liegen dabei in einem Bereich von 1 bis 50 Gew.-%, vorzugsweise in einem Bereich von 5 bis 40 Gew.-% und ganz besonders bevorzugt in einem Bereich von 10 bis 30 Gew.-%. Wird die erfindungsgemäße lipidhaltige Zubereitung als Lichtschutzmittel verwendet, so können ihr Titandioxid und/oder Zinkoxid in Form von sog. "Nanopigmenten" mit Teilchengrößen in Bereich zwischen 5 und 25 nm in einer Menge von 1 bis 20 Gew.-%, vorzugsweise 5 bis 15 Gew.-%, gegebenenfalls auch in Kombination mit üblichen, durch die jeweilige nationale oder regionale Gesetzgebung zugelassene, öllösliche UV-A und/oder UV-B-Lichtfiltersubstanzen zugesetzt werden. Alle diese Mengenangaben für die feste Phase erfolgen ebenfalls mit der Maßgabe, dass sich die Summe aller Inhaltsstoffe der vorgenannten lipidhaltigen Zubereitung auf 100 Gew.-% ergänzt.

Der erfindungsgemäßen Zubereitung können weiterhin noch die für Kosmetika üblichen Zusatzstoffe, wie Duftstoffe, Antioxidanzien, Konservierungsmittel und dgl. in den für die Wirksamkeit notwendig und für kosmetische Massen üblichen Mengen zugegeben werden. Bevorzugt wird dabei auf solche Stoffe zurückgegriffen, die pflanzlichen Ursprungs sind.

Als Färbemittel kommen dabei sowohl anorganische als auch organische Pigmente sowie organische Farbstoffe in Betracht.

Ein weiterer Gegenstand der Erfindung ist auch ein Verfahren zur Herstellung der erfindungsgemäßen wasserfreien lipidhaltigen Zubereitung, wie in den Ansprüchen 30 bis 32 definiert.

Wie oben ausgeführt ist, kann die wasserfreie lipidhaltige Zubereitung aufgrund ihrer besonderen Eigenschaften nicht nur durch Extrudieren sondern auch durch Gießen zu Minen verarbeitet werden. Gegenstand der Erfindung ist daher ein Verfahren zur Herstellung eines Kosmetikstiftes, bei dem die erfindungsgemäße wasserfreie lipidhaltige Zubereitung durch Gießen zu einer Mine geformt wird und dann in an sich bekannter Weise in Holz eingeleimt und zu Stiffen weiterverarbeitet wird.

In einer bevorzugten Ausführungsform wird dabei die wasserfreie lipidhaltige Zubereitung bei erhöhter Temperatur in eine Kunststoffhülse aus spitzbarem Material unter Anformen einer Spitze eingegossen. Verfahren hierzu sind dem Fachmann bekannt.

Aufgrund der besonders vorteilhaften Eigenschaften der erfindungsgemäßen Zubereitung ist es möglich, die daraus gegossenen Minen in einer Drehspindelmechanik zu verwenden. Ein weiterer Gegenstand der Erfindung ist daher auch ein Verfahren zur Herstellung eines Kosmetikstiftes, bei dem die erfindungsgemäße lipidhaltige Zubereitung bei erhöhter Temperatur in eine Drehspindelmechanik eingegossen wird.

Die erfindungsgemäße Zubereitung kann darüber hinaus auch in Form einer Paste in Tuben, Tiegel oder auch Pfännchen abgefüllt werden.

Die Erfindung betrifft wasserfreie lipidhaltige Zubereitungen, insbesondere in Form eines Stiftes oder einer geschmeidigen Paste, die auf die Haut, die Semischleimhäute oder in der Nähe von Schleimhäuten, beispielsweise in der Nähe der Augen, aufgetragen werden. Als Semischleimhäute sollen hier insbesondere die Lippen verstanden werden. Beispielhaft genannt seinen Zubereitungen zum Färben oder zur Pflege der Lippen, Zubereitungen zum Färben oder zur Pflege der Haut, wie beispielsweise Make-up, Rouge, Camouflage zum Kaschieren von Altersflecken oder Rosacea, Conealer, Stifte zum Zeichnen der Lippenkonturen, der Konturen der Augen und der Augenbrauen und ferner Sonnenschutzprodukte mit unterschiedlichen Lichtschutzfaktoren (SPF), bis hin zu sog. Sunblockern unter Verwendung von sehr feinteiligen Nanopigmenten oder bunt eingefärbten, höher pigmentierten Sunblockern, welche beispielsweise als Körperbemalung bei Surfern und Windsurfern beliebt sind.

Die Zubereitung kann auch ohne Zusatz von Färbemitteln hergestellt werden und ggf. sog. kosmetische Wirkstoffe enthalten. Sie findet dann Verwendung als Lipgloss oder als Fixierungsmittel, welche über einen Lippenstift aufgetragen werden. Enthält diese uneingefärbte Zubereitung Lichtschutzfilter, so kann sie als Lippenschutz und -pflege Verwendung finden. Die Haut der Lippen enthält ja bekanntermaßen, im Gegensatz zur Haut des Körpers, keine Pigmentierung. Geeignete öllösliche Lichtfiltersubstanzen, die im Bereich des UV-A und UV-B guten Schutz bieten, sind dem einschlägig befassten Fachmann in ausreichender Zahl bekannt und durch die jeweilige nationale oder regionale Gesetzgebung z.B. in der EU, in Japan und in den U.S.A. geregelt - in Deutschland beispielsweise durch die Anlage 7 zu § 3b der Kosmetik-Verordnung und sollen hier deshalb nicht umfassend aufgelistet werden. Beispielhaft erwähnt seien deshalb nur das Isoamyl p-Methoxycinnamate als UV-B Filter und 4-Methylbenzylidene Camphor als UV-A Filter.

Die erfindungsgemäße Zubereitung soll anhand der nachfolgenden Beispiele im Detail erläutert werden, welche sie jedoch nicht abschließend beschreiben. Dabei erfolgen alle Mengenangaben in Gewichtsprozent (Gew.-%), bezogen auf das Gesamtgewicht der Zubereitung:

### Beispiel 1 - pastenförmiges Lippenrouge

| | |
|---|---|
| Hydrogenated Jojoba Oil | 11,000 |
| Limnanthes Alba | 19,500 |
| Carnauba | 3,500 |
| Buxus Chinensis | 31,550 |
| Butyrosperum Parkii | 3,000 |
| Hydrogenated Cottonseed Oil | 9,500 |
| Pigments | 18,000 |
| Silica | 3,500 |
| Tocopherol | 0,350 |
| Ascorbyl Palmitate | 0,100 |

Die Herstellung erfolgt, indem man Hydrogenated Jojoba Oil, Limnanthes Alba (Meadowfoam Seed Oil), Carnauba, Buxus Chinensis und Hydrogenated Cottonseed Oil in einer geeigneten Homogenisiermaschine mit Ankerrührwerk und Zahnkranzhomogenisator vorlegt und auf etwa 90 °C erwärmt. Anschließend wird das Silica eingestreut und mittels Homogenisator dispergiert. Anschließend werden die Pigmente zugesetzt, die Mischung wird dann unter hohem Scherkrafteintrag homogenisiert, um alle Pigmentagglomerate zu zerstören. Die Masse wird dann durch Anlegen von Vakuum entlüftet. Anschließend werden die Antioxidantien (Tocopherol und Ascorbylpalmitat) zu der noch heißen Mischung zugesetzt, und es wird kurz nachhomogenisiert. Anschließend wird unter Rühren mit dem Ankerrührwerk weiter gekühlt bis etwa 35 °C. Die jetzt pastöse Mischung wird nun in den Abfüllkessel überführt und ohne weitere Maßnahmen auf Raumtemperatur abkühlen lassen. Anschließend wird auf einer Abfüll- oder Montagemaschine in die geeigneten Behältnisse, wie z.B. Tiegel aus Glas oder Kunststoff oder Pfännchen aus Metall abgefüllt, nachdem die Zubereitung von der Qualitätssicherung überprüft und freigegeben wurde. Man erhält eine geschmeidige, weiche Paste mit einer Viskosität von 5.000 mPas.

### Beispiel 2 - Cremelidschatten

| | |
|---|---|
| Candelilla Cera | 2,500 |
| Carnauba | 1,800 |
| Hydrogenated Castor Oil | 3,500 |
| Hydrogenated Jojoba Oil | 9,500 |
| Limnanthes Alba | 18,000 |
| Hydrogenated Cottonseed Oil | 4,000 |
| Buxus Chinensis | 32,250 |
| Pigments | 9,000 |
| Mica (and) Titanium Dioxide | 16,000 |
| Silica | 3,000 |
| Tocopherol | 0,350 |
| Ascorbyl Palmitate | 0,100 |

Die Herstellung erfolgt analog zu Beispiel 1, jedoch werden hierbei zunächst die Pigmente bei etwa 90 °C zu der Fettphase zugegeben und homogen eingearbeitet, danach werden die Perlglanzmittel zugesetzt und der dann nochmals kurz homogenisiert. Man erhält eine weiche, geschmeidige Paste mit schönem Perlglanz und einer Viskosität von 4,200 mPas.

### Beispiel 3 - Lipgloss

| | |
|---|---|
| Carnauba | 1,650 |
| Candelilla Cera | 2,000 |
| Limnanthes Alba | 16,000 |
| Hydrogenated Jojoba Oil | 8,750 |
| Magnifera Indica | 6,000 |
| Macadamia Ternifolia Nut Oil | 7,500 |
| Butyrosperum Parkii | 2,000 |
| Buxus Chinensis | 44,550 |
| Rhus Succedanea | 6,000 |
| Silica | 2,500 |
| Isoamyl p-Methoxycinnamate | 1,500 |
| 4-Methylbenzylidene Camphor | 1,000 |
| Tocopherol | 0,450 |
| Ascorbyl Palmitate | 0,100 |

Die Herstellung erfolgt im wesentlichen analog zu den vorstehend beschriebenen Verfahren, wobei die Lichtfiltersubstanzen zusammen mit dem Tocopherol bei etwa 45-50 °C zugesetzt werden. Man erhält eine ungefärbte, transparente, sehr weiche Paste, mit einer Viskosität von2.800 mPas, welche in Tuben oder Tiegel abgefüllt werden kann.

### Beispiel 4 - Sunblocker für Surfer

| | |
|---|---|
| Carnauba | 3,500 |
| Candelilla Cera | 2,850 |
| Hydrogenated Castör Oil | 4,800 |
| Hydrogenated Vegetable Oil | 7,500 |
| Hydrogenated Jojoba Oil | 9,500 |
| Limnanthes Alba | 16,000 |
| Buxus Chinensis | 23,550 |
| Magnifera Indica | 5,000 |
| Titanium Dioxide (Nanopigment) | 10,000 |
| Iron Oxides (Rot und Gelb) | 6,000 |
| Silica | 3,000 |
| Isoamyl p-Methoxycinnamate | 4,500 |
| 4-Methylbenzylidene Camphor | 3,000 |
| Tocopherol | 1,000 |
| Ascorbyl Palmitate | 0,100 |

Die Herstellung erfolgt nach dem unter Beispiel 1 angegebenen Verfahren. Man erhält eine rotorange feste Zubereitung, die sich vorzugsweise zur Abfüllung in Drehspindel-Mechaniken eignet. Der Lichtschutzfaktor (SPF) dieser Zubereitung liegt oberhalb von 25.

### Beispiel 5 - Lipliner

| | |
|---|---|
| Hydrogenated Jojoba Oil | 34,000 |
| Limnanthes Alba | 20,000 |
| Carnauba | 0,800 |
| Candelilla Cera | 2,500 |
| Magnifera Indica | 4,000 |
| Butyrosperum Parkii | 1,000 |
| Silica | 8,300 |
| Chamomilla Recuita | 1,000 |
| Tocopherol | 0,300 |
| Pigmente | 28,000 |
| Ascorbylpalmitat | 0,100 |

Die Herstellung des Lipliners erfolgt analog Beispiel 1. Die Masse wird in eine Gießmaschine überführt und bei etwa 85-90 °C entweder in Kunststoffhülsen aus einem spitzbaren Material unter Anformen der Spitze eingegossen oder in metallische Gießformen eingegossen und nach dem Erkalten entformt und in bekannter Weise zu holzgefassten Kosmetikstiften weiterverarbeitet.

### Beispiel 6 - Eyeliner

| | |
|---|---|
| Hydrogenated Jojoba Oil | 11,000 |
| Limnanthes Alba | 19,500 |
| Hydrogenated Vegetable Oil | 4,500 |
| Buxus Chinensis | 8,400 |
| Carnauba | 1,700 |
| Candelilla Cera | 2,650 |
| Rhus Succedanea | 4,500 |
| Magnifera Indica | 4,000 |
| Macadamia Ternifolia Nut Oil | 1,900 |
| Pigmente | 30,000 |
| Talc | 6,700 |
| Silica | 3,000 |
| Glyceryl Caprylate | 1,000 |
| Chamomilla Recutita Extract | 0,800 |
| Tocopherol | 0,250 |
| Ascorbylpalmitat | 0,100 |

Die Herstellung des Eyeliners erfolgt analog der unter Beispiel 5 angegebenen Weise. Als Pigmente finden besonders bevorzugt anorganische Pigmente, wie schwarze, rote und gelbe Eisenoxide, Titandioxid, Ferric Blue, Ultramarin, Chromoxidhydratgrün, Chromoxidgrün, Manganviolett und deren Gemische Verwendung. Gegebenenfalls können auch Perlglanzpigmente, wie z.B. Glimmer, mit Metalloxiden beschichtete Glimmer, Bismuthoxychlorid, mit Metalloxiden beschichtetes Bismuthoxychlorid, plättchenförmige Metalle, wie pulverförmiges Aluminium, Bronze, Messing, Titan, Silber, Gold oder Gemische daraus, auch in Kombination mit anorganischen Pigmenten, verwendet werden.

### Beispiel 7 - Augenbrauenstift

| | |
|---|---|
| Hydrogenated Jojoba Oil | 12,500 |
| Limnanthes Alba | 10,500 |
| Rhus Succedanea | 6,000 |
| Hydrogenated Cottonseed Oil | 6,000 |
| Carnauba | 2,200 |
| Candelilla Cera | 2,600 |
| Butyrosperum Parkii | 1,250 |
| Macadamia Ternifolia Nut Oil | 2,200 |
| Buxus Chinensis | 6,300 |
| Pigmente | 35,000 |
| Talc | 12,000 |
| Silica | 3,000 |
| Tocopherol | 0,350 |
| Ascorbylpalmitat | 0,100 |

Die Herstellung erfolgt analog zu Beispiel 1. Nach dem völligen Erkalten wird die Masse noch zweimal über einen Dreiwalzenstuhl passiert, in bekannter Weise kartuschiert und im üblichen Verfahren zu Minen extrudiert, die anschließend in Holzbrettchen eingeleimt und zu fertigen holzgefassten Stiften verarbeitet werden.

Lipidhaltige Zubereitungen, welche keine Wasserphase enthalten, bleiben sehr oft unkonserviert, um - vergleichsweise sehr seltene - Unverträglichkeitsreaktionen von Verbraucherinnen auf Konservierungsmittel möglichst weitgehend auszuschließen. Um jedoch eine Besiedelung der Stiftoberflächen mit Mikroorganismen mit anschließender Rekontamination der Hautoberfläche auszuschließen, können den erfindungsgemäßen lipidhaltigen Zubereitungen pflanzenbasierte Konservierungsmittel, wie z.B. öllöslicher Trockenextrakt von Rosmarin oder Glyceryl Caprate oder Mischungen natürlicher oder naturidentischer Riechstoffe mit antimikrobiellen Eigenschaften, wie z.B. Geraniol, Linalool, Neroli, Vanillin, Eugenol, Methyleugenol, Palmarosaöl und dergleichen in den gewünschten und üblichen Mengen zwischen 0,05 und 1,0 Gew.-% zugesetzt werden. Vorzugsweise wird die notwendige Menge an Konservierungsmittel in einem Konservierungsbelastungstest bestimmt, welcher den einschlägig mit der Thematik befassten Fachleuten hinreichend bekannt ist.

## Patentansprüche

1. Wasserfreie lipidhaltige Zubereitung für kosmetische Anwendungen, welche eine Ölphase und eine Feststoffphase enthält, **dadurch gekennzeichnet, dass** die Ölphase aus einer Mischung von Rohstoffen auf pflanzlicher Basis besteht, die Hydrogenated Jojoba Oil und Limnanthes Alba (Meadowfoam Seed Oil) enthält.

2. Lipidhaltige Zubereitung nach Anspruch 1, **dadurch gekennzeichnet, dass** die pflanzlichen Rohstoffe ausgewählt wurden unter pflanzlichen Ölen, hydrierten pflanzlichen Ölen, pflanzlichen Wachsen und deren Mischungen.

3. Lipidhaltige Zubereitung nach Anspruch 1 oder 2, **dadurch gekennzeichnet, dass** die pflanzlichen Öle ausgewählt sind unter Magnifera Indica (Mango Seed Oil), Macadamia Ternifolia Nut Oil (Macadamia Nut Oil), Butyrosperum Parkii (Shea Butter), Buxus Chinensis (Jojoba Oil) und deren Mischungen.

4. Lipidhaltige Zubereitung nach Anspruch 1 oder 2, **dadurch gekennzeichnet, dass** die hydrierten pflanzlichen Öle ausgewählt sind unter Hydrogenated Cottonseed Oil, Hydrogenated Vegetable Oil, Hydrogenated Castor Oil, Hydrogenated Coco-Glycerides und deren Mischungen

5. Lipidhaltige Zubereitung nach Anspruch 1 oder 2, **dadurch gekennzeichnet, dass** die pflanzlichen Wachse ausgewählt sind unter Carnauba, Candelilla Cera, Rhus Succedanea (Japanwachs), Reiswachs, Zuckerrohrwachs und deren Mischungen.

6. Lipidhaltige Zubereitung nach einem der Ansprüche 1 bis 3, **dadurch gekennzeichnet, dass** der Gehalt an pflanzlichen Ölen in einem Bereich von 1 bis 65 Gew.-%, vorzugsweise in einem Bereich von 5 bis 45 Gew.-% liegt.

7. Lipidhaltige Zubereitung nach einem der Ansprüche 1, 2 oder 5, **dadurch gekennzeichnet, dass** der Gehalt an pflanzlichen Wachsen in einem Bereich von 0,1 bis 30 Gew.-%, vorzugsweise in einem Bereich von 1 bis 20 Gew.-% liegt.

8. Lipidhaltige Zubereitung nach einem der Ansprüche 1, 2 oder 4, **dadurch gekennzeichnet, dass** der Gehalt an hydrierten pflanzlichen Ölen in einem Bereich von 10 bis 80 Gew.-%, vorzugsweise in einem Bereich von 15 bis 50 Gew.-% liegt.

9. Lipidhaltige Zubereitung nach einem der vorhergehenden Ansprüche, **dadurch gekennzeichnet, dass** die Einsatzmenge von Hydrogenated Jojoba Oil und Limnanthes Alba (Meadowfoam Seed Oil) jeweils in einem Bereich zwischen 2 und 35 Gew.-%, vorzugsweise in einem Bereich zwischen 5 und 25 Gew.-% liegt..

10. Lipidhaltige Zubereitung nach einem der vorhergehenden Ansprüche, **dadurch gekennzeichnet, dass** das Verhältnis der Einsatzmengen von Hydrogenated Jojoba Oil zu Limnanthes Alba (Meadowfoam Seed Oil) zwischen 1:2 bis 2:1 liegt.

11. Lipidhaltige Zubereitung nach einem der vorhergehenden Ansprüche, **dadurch gekennzeichnet, dass** sie zusätzlich als Feststoffphase Füllstoffe, anorganische Pigmente, organische Pigmente oder deren Mischungen enthält.

12. Lipidhaltige Zubereitung nach Anspruch 11, **dadurch gekennzeichnet, dass** es sich bei den Füllstoffen um Talkum, Kaolin, Stärke, modifizierte Stärke, Polytetrafluorethylenpulver, Nylonpulver, Bornitrid, Mg-Stearat, Ca-Stearat, Sr-Stearat, Zn-Stearat oder deren Mischungen handelt.

13. Lipidhaltige Zubereitung nach einem der Ansprüche 11 oder 12, **dadurch gekennzeichnet, dass** es sich bei den anorganischen Pigmenten um Titandioxid, Zinkoxid, Eisenoxide, Chromoxid, Chromoxidhydrat, Ultramarin, Berliner Blau (Ferric Blue), Glimmer, mit Titandioxid beschichtete Glimmer, mit Titandioxid und mit Metalloxiden beschichtete Glimmer, Bismuthoxidchlorid, beschichtetes Bismuthoxidchlorid, plättchenförmige Metallpulver von Aluminium, Messing, Bronze, Kupfer, Silber, Gold oder deren Mischungen handelt.

14. Lipidhaltige Zubereitung nach einem der Ansprüche 11 bis 13, **dadurch gekennzeichnet, dass** es sich bei den organischen Pigmenten um Verlackungen organischer Färbemittel mit Aluminium, Barium, Calcium, Strontium, Zirkon und um deren Mischungen handelt.

15. Lipidhaltige Zubereitung nach einem der Ansprüche 11 bis 14, **dadurch gekennzeichnet, dass** die Mengenanteile an Pigmenten in einem Bereich von 1 bis 50 Gew.-%, bevorzugt in einem Mengenbereich von 5 bis 40 Gew.-%, ganz besonders bevorzugt in einem Bereich von 10 bis 30 Gew.-% liegen.

16. Lipidhaltige Zubereitung nach einem der Ansprüche 11 bis 15, **dadurch gekennzeichnet, dass** Nanopigmente mit Teilchengrößen im Bereich von 5 bis 25 nm verwendet werden.

17. Lipidhaltige Zubereitung nach einem der Ansprüche 11 bis 16, **dadurch gekennzeichnet, dass** das als Lichtschutzmittel verwendete Titandioxid und/oder Zinkoxid in Form sog. Nanopigmente in einer Menge von 5 bis 25 Gew.-%, vorzugsweise in einer Menge von 5 bis 15 Gew.-%, eingesetzt wird

18. Lipidhaltige Zubereitung nach einem der Ansprüche 11 bis 17, **dadurch gekennzeichnet, dass** die sog. Nanopigmente gegebenenfalls in Kombination mit üblichen, öllöslichen UV-A- und UV-B-Lichtfiltersubstanzen verwendet werden.

19. Lipidhaltige Zubereitung nach einem der Ansprüche 11 bis 18, **dadurch gekennzeichnet, dass** in Kombination mit sog. Nanopigmenten als UV-A-und UV-B-Lichtfiltersubstanzen bevorzugt 4-Methylbenzylidene Camphor und Isoamyl p-Methoxycinnamate verwendet werden.

20. Lipidhaltige Zubereitung nach einem der vorhergehenden Ansprüche, **dadurch gekennzeichnet, dass** sie, als Mittel zur Pflege der Haut und/oder als Mittel aus dem Bereich der dekorativen Kosmetik zum Färben und Verschönen der Haut, der Lippen und/oder der Augenlider vorliegt.

21. Lipidhaltige Zubereitung nach einem der vorhergehenden Ansprüche, **dadurch gekennzeichnet, dass** sie als Lippenrouge, Wangenrouge, Lipliner, Eyeliner, Augenbrauenstift, Lidschatten, Make-up, Abdeckstift, Concealer, Lippenpflegestift, Lippenbalsam, Lipgloss, fixierende Grundlage für die Lippen, Pflegegrundlage für die Haut oder als Sonnenschutzmittel vorliegt.

22. Lipidhaltige Zubereitung nach einem der vorhergehenden Ansprüche, **dadurch gekennzeichnet, dass** sie ein Konservierungsmittel, wobei das Konservierungsmittel ein pflanzenbasiertes Konservierungsmittel ist, und/oder natürliche und/oder naturidentische Riechstoffe oder Mischungen daraus enthält.

23. Lipidhaltige Zubereitung nach Anspruch 22, **dadurch gekennzeichnet, dass** die Einsatzmenge an pflanzenbasiertem Konservierungsmittel und/oder natürlichen und/oder naturidentischen Riechstoffen oder Mischungen daraus 0,05 bis 1,0 Gew.-% beträgt.

24. Lipidhaltige Zubereitung nach einem der vorhergehenden Ansprüche, **dadurch gekennzeichnet, dass** sie als Konservierungsmittel einen öllöslichen Trockenextrakt von Rosmarin oder Glyceryl Caprate oder eine Mischung daraus enthält.

25. Lipidhaltige Zubereitung nach einem der vorhergehenden Ansprüche, **dadurch gekennzeichnet, dass** sie als Konservierungsmittel natürliche oder naturidentische Riechstoffe oder Mischungen daraus enthält, die ausgewählt wurden unter Geraniol, Linalool, Neroli, Vanillin, Eugenol, Methyleugenol oder Palmarosaöl.

26. Holzstift mit einer Mine, wobei die Mine aus einer lipidhaltigen Zubereitung nach einem der vorhergehenden Ansprüche geformt ist.

27. Stift mit einer Kunststoffhülse aus spitzbarem Material und einer Mine, wobei die Mine aus einer lipidhaltigen Zubereitung nach einem der vorhergehenden Ansprüche gegossen wurde.

28. Stift mit Drehspindelmechanik und darin eingesetzter Mine, wobei die Mine aus einer lipidhaltigen Zubereitung nach einem der vorhergehenden Ansprüche gegossen wurde.

29. Behälter ausgewählt aus einer Tube, einem Tiegel oder einem Metallpfännchen und darin enthaltenener Paste, wobei die Paste eine lipidhaltige Zubereitung nach einem der vorhergehenden Ansprüche ist.

30. Verfahren zur Herstellung eines Kosmetikstifts, wobei eine Zubereitung wie in einem der vorhergehenden Ansprüche definiert durch Gießen zu einer Mine geformt wird und in an sich bekannter Weise zu einem Stift weiterverarbeitet wird.

31. Verfahren nach Anspruch 30, wobei die durch Gießen geformte Mine in eine Drehmechanik eingesetzt wird.

32. Verfahren nach Anspruch 30, wobei die durch Gießen geformte Mine in Holz eingelegt, verleimt und zu einem Stift verarbeitet wird.

## Claims

1. An anhydrous lipid-containing preparation for cosmetic applications that contains an oil phase and a solid phase, **characterised in that** the oil phase consists of a mixture of vegetable-based raw materials containing hydrogenated jojoba oil and *Limnanthes alba* (meadowfoam seed oil).

2. A lipid-containing preparation according to claim 1, **characterised in that** the vegetable raw materials have been selected from vegetable oils, hydrogenated vegetable oils, vegetable waxes and mixtures thereof.

3. A lipid-containing preparation according to claim 1 or 2, **characterised in that** the vegetable oils are selected from *Mangifera indica* (mango seed oil), *Macadamia ternifolia* nut oil (macadamia nut oil), *Butyrospermum parkii* (shea butter), *Buxus chinensis* (jojoba oil) and mixtures thereof.

4. A lipid-containing preparation according to claim 1 or 2, **characterised in that** the hydrogenated vegetable oils are selected from hydrogenated cottonseed oil, hydrogenated vegetable oil, hydrogenated castor oil, hydrogenated coco-glycerides and mixtures thereof.

5. A lipid-containing preparation according to claim 1 or 2, **characterised in that** the vegetable waxes are selected from carnauba, candelilla cera, *Rhus succedanea* (Japan wax), rice wax, sugar-cane wax and mixtures thereof.

6. A lipid-containing preparation according to one of claims 1 to 3, **characterised in that** the content of vegetable oils lies in a range of 1 to 65 % by weight, preferably in a range of 5 to 45 % by weight.

7. A lipid-containing preparation according to one of claims 1, 2 or 5, **characterised in that** the content of vegetable waxes lies in a range of 0.1 to 30 % by weight, preferably in a range of 1 to 20 % by weight.

8. A lipid-containing preparation according to one of claims 1, 2 or 4, **characterised in that** the content of hydrogenated vegetable oils lies in a range of 10 to 80 % by weight, preferably in a range of 15 to 50 % by weight.

9. A lipid-containing preparation according to one of the preceding claims, **characterised in that** the quantity of hydrogenated jojoba oil and *Limnanthes alba* (meadowfoam seed oil) employed in each case lies in a range between 2 and 35 % by weight, preferably in a range between 5 and 25 % by weight.

10. A lipid-containing preparation according to one of the preceding claims, **characterised in that** the ratio of the quantities of hydrogenated jojoba oil to *Limnanthes alba* (meadowfoam seed oil) employed lies between 1:2 to 2:1.

11. A lipid-containing preparation according to one of the preceding claims, **characterised in that** it additionally contains as the solid phase fillers, inorganic pigments, organic pigments or mixtures thereof.

12. A lipid-containing preparation according to claim 11, **characterised in that** the fillers are talcum, kaolin, starch, modified starch, polytetrafluoroethylene powder, nylon powder, boron nitride, Mg stearate, Ca stearate, Sr stearate, Zn stearate or mixtures thereof.

13. A lipid-containing preparation according to one of claims 11 or 12, **characterised in that** the inorganic pigments are titanium dioxide, zinc oxide, iron oxides, chromium oxide, chromium hydroxide, ultramarine, Berlin blue (ferric blue), mica, mica coated with titanium dioxide, mica coated with titanium dioxide and with metal oxides, bismuth oxide chloride, coated bismuth oxide chloride, metal powder in flake form of aluminium, brass, bronze, copper, silver, gold or mixtures thereof.

14. A lipid-containing preparation according to one of claims 11 to 13, **characterised in that** the organic pigments are means for producing lakes of organic colourants with aluminium, barium, calcium, strontium, zirconium and mixtures thereof.

15. A lipid-containing preparation according to one of claims 11 to 14, **characterised in that** the quantitative proportions of pigments lie in a range of 1 to 50 % by weight, preferably in a quantitative range of 5 to 40 % by weight, more particularly preferably in a range of 10 to 30 % by weight.

16. A lipid-containing preparation according to one of claims 11 to 15, **characterised in that** nanopigments having particle sizes in the range of 5 to 25 nm are used.

17. A lipid-containing preparation according to one of claims 11 to 16, **characterised in that** the titanium dioxide and/or zinc oxide used as a light-protection agent in the form of so-called nanopigments is employed in a quantity of 5 to 25 % by weight, preferably in a quantity of 5 to 15 % by weight.

18. A lipid-containing preparation according to one of claims 11 to 17, **characterised in that** the so-called nanopigments are used, if applicable, in combination with standard oil-soluble UV-A- and UV-B-light-filter substances.

19. A lipid-containing preparation according to one of claims 11 to 18, **characterised in that** preferably 4-methylbenzylidene camphor and isoamyl p-methoxycinnamate are used in combination with so-called nanopigments as UV-A- and UV-B-light-filter substances.

20. A lipid-containing preparation according to one of the preceding claims, **characterised in that** it is provided as an agent to care for the skin and/or as an agent from the range of decorative cosmetics to colour and beautify the skin, the lips and/or the eyelids.

21. A lipid-containing preparation according to one of the preceding claims, **characterised in that** it is provided as a lip-rouge, blusher, lip-liner, eye-liner, eyebrow-pencil, eye-shadow, make-up, cover-up stick, concealer, lip-care stick, lip-balm, lip-gloss, fixing foundation for the lips, care foundation for the skin or as a sun-protection agent.

22. A lipid-containing preparation according to one of the preceding claims, **characterised in that** it contains a preserving agent, wherein the preserving agent is a vegetable-based preserving agent, and/or natural perfumes and/or nature-identical perfumes or mixtures thereof.

23. A lipid-containing preparation according to claim 22, **characterised in that** the quantity of vegetable-based preserving agent and/or natural and/or nature-identical perfumes or mixtures thereof employed amounts to 0.05 to 1.0 % by weight.

24. A lipid-containing preparation according to one of the preceding claims, **characterised in that** it contains as preserving agent an oil-soluble dry extract of rosemary or glyceryl caprate or a mixture thereof.

25. A lipid-containing preparation according to one of the preceding claims, **characterised in that** it contains as preserving agents natural or nature-identical perfumes or mixtures thereof selected from geraniol, linalool, neroli, vanillin, eugenol, methyleugenol or palmarosa oil.

26. A wooden pencil having a lead, wherein the lead is formed from a lipid-containing preparation according to one of the preceding claims.

27. A pencil having a plastics sleeve made from material that can be sharpened and a lead, wherein the lead has been cast from a lipid-containing preparation according to one of the preceding claims.

28. A pencil having a rotary spindle mechanism and a lead inserted therein, wherein the lead has been cast from a lipid-containing preparation according to one of the preceding claims.

29. A container selected from a tube, a jar or a small metal pot and paste contained therein, wherein the paste is a lipid-containing preparation according to one of the preceding claims.

30. A method for producing a cosmetic pencil, wherein a preparation as defined in one of the preceding claims is shaped by casting to form a lead and processed further in a manner known per se to form a pencil.

31. A method according to claim 30, wherein the lead shaped by casting is inserted into a rotary mechanism.

32. A method according to claim 30, wherein the lead shaped by casting is laid in wood, glued and processed to form a pencil.

## Revendications

1. Préparation lipidique anhydre pour applications cosmétiques, qui contient une phase huileuse et une phase solide, **caractérisée en ce que** la phase huileuse se compose d'un mélange de matières brutes à base végétale qui contient de l'huile de jojoba hydrogénée et Limnanthes Alba (huile de graine de limnanthe).

2. Préparation lipidique selon la revendication 1, **caractérisée en ce que** les matières brutes végétales ont été sélectionnées parmi des huiles végétales, des huiles végétales hydrogénées, des cires végétales et leurs mélanges.

3. Préparation lipidique selon la revendication 1 ou 2, **caractérisée en ce que** les huiles végétales sont sélectionnées parmi Magnifera Indica (huile de graine de mangue), Macadamia Ternifolia Nut Oil (huile de noix de macadamia), Butyrosperum Parkii (beurre de karité), Buxus Chinensis (huile de jojoba) et leurs mélanges.

4. Préparation lipidique selon la revendication 1 ou 2, **caractérisée en ce que** les huiles végétales hydrogénées sont sélectionnées parmi l'huile de graine de coton hydrogénée, l'huile végétale hydrogénée, l'huile de ricin hydrogénée, des coco-glycérides hydrogénés et leurs mélanges.

5. Préparation lipidique selon la revendication 1 ou 2, **caractérisée en ce que** les cires végétales sont sélectionnées parmi le cardauba, Candelilla Cera, Rhus Succedanea (cire du Japon), la cire de riz, la cire de canne à sucre et leurs mélanges.

6. Préparation lipidique selon une des revendications 1 à 3, **caractérisée en ce que** la teneur en huiles végétales se situe dans une plage de 1 à 65 % en poids, de préférence dans une plage de 5 à 45 % en poids.

7. Préparation lipidique selon une des revendications 1, 2 ou 5, **caractérisée en ce que** la teneur en cires végétales se situe dans une plage de 0,1 à 30 % en poids, de préférence dans une plage de 1 à 20 % en poids.

8. Préparation lipidique selon une des revendications 1, 2 ou 4, **caractérisée en ce que** la teneur en huiles végétales hydrogénées se situe dans une plage de 10 à 80 % en poids, de préférence dans une plage de 15 à 50 % en poids.

9. Préparation lipidique selon une des revendications précédentes, **caractérisée en ce que** la quantité totale d'huile de jojoba hydrogénée et de Limnanthes Alba (huile de graine de limnanthe) se situe à chaque fois dans une plage entre 2 et 35 % en poids, de préférence dans une plage entre 5 et 25 % en poids.

10. Préparation lipidique selon une des revendications précédentes, **caractérisée en ce que** le rapport des quantités totales d'huile de jojoba hydrogénée sur Limnanthes Alba (huile de graine de limnanthe) se situe entre 1/2 et 2/1.

11. Préparation lipidique selon une des revendications précédentes, **caractérisée en ce qu'**elle contient en outre comme phase solide des charges, pigments inorganiques, pigments organiques ou leurs mélanges.

12. Préparation lipidique selon la revendication 11, **caractérisée en ce qu'**il s'agit pour les charges de talc, kaolin, amidon, amidon modifié, poudre de polytétrafluoroéthylène, poudre de nylon, nitrure de bore, stéarate de Mg, stéarate de Ca, stéarate de Sr, stéarate de Zn ou leurs mélanges.

13. Préparation lipidique selon une des revendications 11 ou 12, **caractérisée en ce qu'**il s'agit pour les pigments inorganiques de dioxyde de titane, oxyde de zinc, oxydes de fer, oxyde de chrome, oxyde de chrome hydraté, bleu outremer, bleu de Prusse (bleu ferrique), mica, mica enduit de dioxyde de titane, mica enduit de dioxyde de titane et d'oxydes métalliques, chloroxyde de bismuth, chloroxyde de bismuth enduit, poudre métallique en forme de paillette d'aluminium, laiton, bronze, cuivre, argent, or ou leurs mélanges.

14. Préparation lipidique selon une des revendications 11 à 13, **caractérisée en ce qu'**il s'agit pour les pigments organiques de formations de pigments de colorants organiques avec de l'aluminium, du baryum, du calcium, du strontium, du zirconium et de leurs mélanges.

15. Préparation lipidique selon une des revendications 11 à 14, **caractérisée en ce que** les proportions quantitatives de pigments se situent dans une plage de 1 à 50 % en poids, de préférence dans une plage quantitative de 5 à 40 % en poids, de manière tout particulièrement préférée dans une plage de 10 à 30 % en poids.

16. Préparation lipidique selon une des revendications 11 à 15, **caractérisée en ce que** des nanopigments avec des tailles particulaires dans la plage de 5 à 25 nm sont utilisés.

17. Préparation lipidique selon une des revendications 11 à 16, **caractérisée en ce que** le dioxyde de titane et/ou l'oxyde de zinc utilisé comme agent photoprotecteur est utilisé sous la forme de ce qu'on appelle des nanopigments en une quantité de 5 à 25 % en poids, de préférence en une quantité de 5 à 15 % en poids.

18. Préparation lipidique selon une des revendications 11 à 17, **caractérisée en ce que** ce qu'on appelle les nanopigments sont éventuellement utilisés en combinaison avec des substances de filtration de lumière UV-A et UV-B usuelles, oléosolubles.

19. Préparation lipidique selon une des revendications 11 à 18, **caractérisée en ce que** du 4-méthylbenzylidène camphre et des p-méthoxycinnamates isoamyliques sont de préférence utilisés en combinaison avec ce qu'on appelle des nanopigments comme substances de filtration de lumière UV-A et UV-B.

20. Préparation lipidique selon une des revendications précédentes, **caractérisée en ce qu'**elle est présente comme agent de soin de la peau et/ou comme agent provenant du domaine de la cosmétique décorative pour la coloration et l'embellissement de la peau, des lèvres et/ou des paupières.

21. Préparation lipidique selon une des revendications précédentes, **caractérisée en ce qu'**elle est présente comme rouge à lèvres, rouge aux joues, crayon à lèvres, eyeliner, crayon pour sourcils, fard à paupières, maquillage, crayon correcteur, crayon concealer, crayon de soin pour les lèvres, baume pour les lèvres, brillant à lèvres, base fixatrice pour les lèvres, base de soin pour la peau ou comme agent de protection solaire.

22. Préparation lipidique selon une des revendications précédentes, **caractérisée en ce qu'**elle contient un conservateur, dans laquelle le conservateur est un conservateur à base de plantes, et/ou des parfums naturels et/ou identiques aux naturels ou mélanges de ceux-ci.

23. Préparation lipidique selon la revendication 22, **caractérisée en ce que** la quantité totale de conservateur à base de plantes et/ou de parfums naturels et/ou identiques aux naturels ou mélanges de ceux-ci se monte à 0,05 à 1,0 % en poids.

24. Préparation lipidique selon une des revendications précédentes, **caractérisée en ce qu'**elle contient comme conservateur un extrait sec oléosoluble de romarin ou de caprate de glycérol ou un mélange de ceux-ci.

25. Préparation lipidique selon une des revendications précédentes, **caractérisée en ce qu'**elle contient comme conservateur des parfums naturels ou identiques aux naturels ou mélanges de ceux-ci qui ont été sélectionnés parmi le géraniol, linalool, néroli, la vanilline, l'eugénol, le méthyleugénol ou l'huile de Palmarosa.

26. Crayon en bois avec une mine, dans lequel la mine est moulée à partir d'une préparation lipidique selon une des revendications précédentes.

27. Crayon avec un manchon plastique en matière pouvant être taillée et une mine, dans lequel la mine est coulée à partir d'une préparation lipidique selon une des revendications précédentes.

28. Crayon avec mécanisme de broche de tournage et mine insérée en son sein, dans lequel la mine est coulée à partir d'une préparation lipidique selon une des revendications précédentes.

29. Récipient sélectionné parmi un tube, un creuset ou un poêlon métallique et pâte contenue en son sein, dans lequel la pâte est une préparation lipidique selon une des revendications précédentes.

30. Procédé de fabrication d'un crayon cosmétique, dans lequel une préparation comme définie dans une des revendications précédentes est moulée en une mine par coulage et est transformée davantage en un crayon d'une manière connue en soi.

31. Procédé selon la revendication 30, dans lequel la mine moulée par coulage est insérée dans un mécanisme rotatif.

32. Procédé selon la revendication 30, dans lequel la mine moulée par coulage est mise en place dans du bois, collée et transformée en un crayon.
